Europäisches Patentamt

European Patent Office

Office européen des brevets

(11) Publication number: **0 415 801 A1**

## EUROPEAN PATENT APPLICATION

(21) Application number: 90401836.3

(22) Date of filing: 26.06.90

(51) Int. Cl.⁵: **C12P 21/08**, G01N 33/577, //C12N15/12,C12Q1/68

The title of the invention has been amended (Guidelines for Examination in the EPO, A-III, 7.3).

(30) Priority: 05.07.89 EP 89401932

(43) Date of publication of application: 06.03.91 Bulletin 91/10

(84) Designated Contracting States: AT BE CH DE DK ES FR GB GR IT LI LU NL SE

(71) Applicant: N.V. INNOGENETICS S.A. Industriepark Zwijnaarde 7, Box 4 B-9710 Gent(BE)

(72) Inventor: Gheuens, Jan Cottagelaan 43 B-8458 Oostduinkerke(BE) Inventor: Van Heuverswyn, Hugo Colmanstraat 62 B-9288 Laarne(BE)

(74) Representative: Grosset-Fournier, Chantal Catherine et al ERNEST GUTMANN-YVES PLASSERAUD S.A., 67 boulevard Haussmann F-75008 Paris(FR)

(54) Monoclonal antibodies against activated microglial cells.

(57) The invention relates to a monoclonal antibody which has the combination of the following properties:
- it forms an immunological complex with a non-phosphorylated epitope of an antigen belonging to activated microglial cells of the central nervous system and released from a sonicated NFT preparation, itself isolated from the cerebral cortex obtained from a patient having Alzheimer's disease,
- it forms an immunological complex with histiocytes and macrophages of the central nervous system, said monoclonal antibody can be used for the diagnosis in vitro of brain diseases involving activated microglial cells.

# MONOCLONAL ANTIBODIES DIRECTED AGAINST ACTIVATED MICROGLIA CELLS, HYBRIDOMAS SECRETING THESE MONOCLONAL ANTIBODIES, ANTIGEN RECOGNIZED BY THESE MONOCLONAL ANTIBODIES AND THEIR APPLICATIONS

The invention relates to new monoclonal antibodies involved in Alzheimer's disease, and to hybridomas secreting such monoclonal antibodies. It is also relative to an antigen which forms an immunological complex with one of said monoclonal antibodies. The invention also relates to a process for the diagnosis in vitro of brain diseases or brain infections, involving activated microglial cells.

The microglial cells are involved in the normal and pathological nervous system but their identification as well as their role is not yet known.

In fact, little is known about microglia. Since its description by Del Rio Hortega in Bol Soc Esp Biol 9, 69-120, its mere existence has remained a controversy. Some authors maintain that microglia is a specialized cell line resident in brain tissue, which differentiates into macrophages upon appropriate stimulation. For a more complete disclosure of this, one can refer particularly to :
- Boya J.J. Calvo A. Carbonell, E. Garcia-Maurino (1986) Nature of macrophages in rat brain, Acta Anat 127, 142-145,
- Boya J., J. Calvo, A.L. Carbonell (1987) Appearance of microglial cells in the postnatal rat retina. Arch. Histol Jap 50, 223-228
- Fujimoto E., A. Miki, H. Mizoguti (1987) Histochemical studies of the differentiation of microglial cells in the cerebral hemispheres of chick embryos and chicks. Histochem 87: 209-216
- Fujita S. (1980) Cytogenesis and pathology of neuroglia and microglia. Pathol Res Pract 168 : 271-278).

Other authors state that all macrophages in brain lesions are hematogenic (Hickey W.F., H. Kimura (1988) Perivascular microglia cells of the CNS are bone marrow-derived and present antigen in vivo. Science 239, 290-292). Classically, the microglial cell is described as a small cell with delicate ramifications, arising at nearly right angles (Dolman C., in Davis and Robertson, Textbook of Neuropathology). In some diseases microglia becomes elongated and gives rise to rod cells. In encephalitis, together with lymphocytes, they constitute microglial nodules. Pronounced microglial proliferation has been recognized in and around senile plaques in Alzheimer's disease (Terry R.D (1985), Alzheimer's disease in : R.L. Davis and D.M. Robertson, Textbook of Neuropathology, Williams and Wilkins, Baltimore pp. 824-841 - Terry R.D. Wisneiwski H.M. (1970) The ultrastructure of the neurofibrillary tangle and the senile plaque. In : Wolstenholme G. and O'Connor M. (eds) Ciba Foundation Symposium on Alzheimer's disease and related conditions).

The identification of microglial cells and assessment of their role in the normal and pathological nervous system has been hampered by lack of a specific staining technique. The silver impregnation technique described by Del Rio Hortega not only stains microglia but also oligodendroglia and some astrocytes (Ganter et J Jollès (1969) Histochimie. Gauthier Villars, Paris, pp. 1463-1466). Microglia can be immunolabelled with different antibodies, some of which also react with circulating macrophages (Esiri M. M., J. Boss (1984) Comparison of methods to identify microglial cells and macrophages in the human central nervous system. J. Clin Pathol 37, 150-156 - Vazeux R., N. Brousse, A. Jarry, D. Henin, C. Marche, C. Vedrenne, J. Mikol, M. Wolff, C. Michon, W. Rozenbaum, J.F. Bureau, L. Montagnier, M. Brahic, (1987) AIDS subacute encephalitis. Identification of HIV-infected cells. Am J Pathol 126, 403-410). However, these antibodies are not specific to microglial cells, and they can only be applied on cryosections, with exception of some anti-HLA-DR antibodies that can be used on paraffin embedded material (Vazeux, see above mentioned reference). Microglia and macrophages have also been stained with antibodies directed to $\alpha_1$-antichymotrypsin, lysozyme and $\alpha_1$-antitrypsin (Esiri, see above mentioned reference). Some reactive cells of possible microglial origin can be demonstrated in the central nervous system with histochemical stainings for acid phosphatase and non-specific esterase (Bancroft J.D. (1975) Histochemical techniques. 2nd ed London, Butterworth, p. 254). Recently, Ricinus communis agglutinin type-1 (RCA-1) has been used to stain cells with the morphology of microglia (Mannoji H., H. Yeger, L.E. Becker (1986) A specific histochemical marker (lectin Ricinus communis agglutinin-1) for normal human microglia and application to routine histopathology. Acta Neuropathol (Berl) 71, 341-343). This lectin, specific for a lactose moiety, can be used on paraffin embedded formalin fixed material. However, RCA-1 staining is not specific for microglia, since endothelial cells are also stained.

Up to now, there was no way of specifically detecting microglial cells, and in particular to distinguish them from other cells such as oligodendroglia, astrocytes and endothelial cells.

The aim of the invention is to provide with monoclonal antibodies which enable to specifically detect activated microglial cells.

The invention also provides with hybridomas secreting the above said monoclonal antibodies.

The invention provides with an antigen which is expressed in a subpopulation of microglial cells reactive to various pathologic conditions.

The invention provides with a process for the detection or diagnosis in vitro of brain diseases involving activated microglial cells, e.g. brain tumors, brain infections, AIDS, Alzheimer's disease.

A monoclonal antibody of the invention is characterized by the fact that it forms an immunological complex with the syngeneic polyclonal anti-idiotypic serum raised against the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG).

A monoclonal antibody of the invention is characterized by the fact that it forms an immunological complex with the monoclonal anti-idiotypic antibody raised against the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG).

The monoclonal antibodies of the invention are defined through their idiotype. Idiotypes are sets of idiotopes, i.e. a collection of individually specific antigenic determinants of immunoglobulins (in contrast to allotype and isotype), and can therefore be considered to be a "fingerprint" of an antibody. For full disclosure of idiotype and anti-idiotype, see e.g. de Préval C: Immunoglobulins, p. 144-219 in Bach J.F.: Immunology, Publ. Wiley and Sons, new York, 1978 or Fleischmann J.B., Davie J.M.: Immunoglobulins: allotypes and idiotypes, pp. 205-220 in Paul W.E.:Fundamental Immunology, Publ. Raven Press, New York, 1984. The monoclonal antibodies of the invention specifically react with the anti-idiotypic serum raised against the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG), in BALB/c mice in syngeneic conditions. Other BALB/c monoclonal antibodies, of whichever class, subclass or type, fail to react with said syngeneic serum to the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG), thus demonstrating the idiotype-specific character of the syngeneic antiserum.

The syngeneic polyclonal anti-idiotypic serum raised against the monoclonal antibody of the invention is obtained by immunization of an animal with a monoclonal antibody of the invention which is raised in a syngeneic, i.e. genetically identical animal, said syngeneic polyclonal serum raised against a monoclonal antibody of the invention containing no other anti-immunoglobulin antibodies than the anti-idiotypic antibodies.

The syngeneic polyclonal anti-idiotypic serum raised against a monoclonal antibody of the invention enables to identify said monoclonal antibody, particularly because said syngeneic polyclonal anti-idiotypic serum does not select for public idiotopes and contains high titers of anti-idiotypic antibodies to private idiotopes of the monoclonal antibodies and particularly because said syngeneic polyclonal anti-idiotypic serum contains and defines the whole set of idiotopes.

The methods used for production of syngeneic anti-idiotypic serum to monoclonal antibodies have been described before (Gheuens J., mcfarlin D.E., Rammohan K.W., Bellini W.J.: Idiotypes and biological activity of murine monoclonal antibodies against the hemagglutinin of measles virus, Inf. Immun. 34: 200-207, 1981; Bona C., Hooghe R., Cazenave P.A., Leguern C., Paul W.E.: Cellular basis of regulation of expression of idiotype II. Immunity to anti-MOPC460 iditoype antibodies increases the level of anti-trinitro-phenyl-antibodies bearing the 460 idiotypes. J. Exp. Med. 149:815-823, 1979).

The methods for production of monoclonal antiidiotypic antibodies have been fully described (Gheuens J., MacFarlin D.E.: Use of monoclonal anti-idiotypic antibody to P3-X63Ag8 myeloma protein for analysis and purification of B lymphocyte hybridoma products. Eur. J. Immunol. 12:701-703, 1982).

A monoclonal antibody according to the invention is defined by the combination of the following properties:
- it forms an immunological complex with a non-phosphorylated structural epitope of an antigen belonging to activated microglial cells of the central nervous system and released from a sonicated NFT preparation, itself isolated from the cerebral cortex obtained from a patient having Alzheimer's disease,
- it forms an immunological complex with histiocytes and macrophages in the vicinity of necrosis areas in the central nervous system.

The expression "structural epitope" refers to an epitope defined by the primary structure, but not the conformation of the antigen. In other words, this epitope is preserved even after treatment of the antigen in ways that will alter its conformation, such as fixation of the antigen with formalin, glutaraldehyde or paraformaldehyde, and after denaturation of the antigen preparation with ionic and non-ionic detergents.

The expression "non phosphorylated epitope" means that the epitope of the antigen to which the monoclonal antibodies of the invention bind are not phosphorylated, as determined by the following test, which comprises :
- starting from an NFT-enriched fraction prepared as described (Iqbal K, Zaidi T, Thompson CH, et al. Alzheimer paired helical filaments: bulk isolation, solubility and protein composition. Acta Neuropath. 1984; 62:167-177), immunoblotted to nitrocellulose as described above and treated overnight at 37°C with 1 IU of Type III E.coli alkaline-phosphatase, in 100ml 0.1M Tris buffer, pH8.0, containing 0.01M phenyl-methyl-sulfonyl-fluoride, applying one of the monoclonal antibodies of the invention to said NFT, forming an immunological complex between NFT and the monoclonal antibody.

The expression "activated microglial cells" refers to a microglial cell that has differentiated from the "resting" state to an "active" state under the influence of a pathological process in its vicinity. The precise nature of the cell biological processes that comprise this transition from "resting" to "active" are not yet defined, and the term "activated microglial cell" hence is a purely operational definition at this time.

The expression "antigen belonging to activated microglial cells" means antigen expressed in microglial cells in the vicinity of a pathological process in the central nervous system, or in microglial cells that participate in a systemic pathological process, but not expressed in microglial cells in the normal central nervous system.

The expression "form an immunologically complex with" means that the monoclonal antibody of the invention binds to the abovesaid antigen under one in the following conditions in the following techniques.

- Light immunomicroscopy:

Brain tissue sample, obtained at surgery or autopsy, was fixed by immersion in 4% formalin or Bouin's fixative and embedded in paraffin. Four mm thick sections were prepared. The monoclonal antibody of the invention was applied either with the avidinbiotinylated peroxidase complex technique (Hsu SM, Raine L, Fanger H. Use of avidin-biotin complex (ABC) in immunoperoxydase techniques. J. Histochem. Cytochem. 1981; 29:577-580), or with the soluble peroxidase-anti-peroxidase complex technique (Sternberger LA, Immunocytochemistry (3rd ed.). Wiley, New York, 1986).

Diaminobenzidine was used as chromogen.

- Immunoelectron microscopy in tissue sections:

Brain tissue sample, obtained at surgery or autopsy is fixed in either Bouin's fixative or 10% buffered formalin before sectioning 60mm thick without embedding (Vibratome). The sections were immunostained by the indirect immunogold method, fixed, embedded and sectioned for electronmicroscopy as described (Perry G, Mulvihill P, Manetto V, et al. Immunocytochemical properties of Alzheimer straight filaments. J. Neurosci. 1987; 7:3736:3738).

- Immunoblotting procedures:

Fractions enriched in PHF, prepared as described (Igbal K, Zaidi T, Thompson CH, et al. Alzheimer paired helical filaments: bulk isolation, solubility and protein composition. Acta Neuropath. 1984; 62:167-177) were sonicated and used as samples in SDS-polyacrylamide electrophoresis and immunoblots. SDS-polyacrylamide electrophoresis was done under reducing conditions on 12% gels (Laemmli UK. Cleavage of structural proteins during the assembly of bacteriophage T4. Nature 1970;227:680-685). After electrophoresis, the proteins were either fixed and stained with Coommassie brilliant blue, or transferred (Towbin H, Staehelin T, Gordon J. Electrophoretic transfer of proteins from polyacrylamide gels to nitrocellulose sheets: procedure and some applications. Proc. Natl. Acad. Sci. USA 1979;76:4350-4354) to nitrocellulose (Hybond-C, Amersham) or Immobilon filters (Millipore). After transfer the filters were presoaked in PBS containing 0.05% (v/v) Tween 20 (Tween-PBS) and then incubated for 1h in Tween-PBS containing 5% (w/v) skimmed dried milk and 5% (v/v) normal goat serum (blocking buffer). Next, the filters were treated overnight at 4°C with primary antibody appropriately diluted in blocking buffer. The filters were then washed three times in Tween-PBS and treated for 1 1/2h at room temperature with biotinylated goat anti-mouse IgM (Amersham) diluted 1/250 in blocking buffer. After three washes in Tween-PBS, streptavidine-biotinylated horseradish peroxidase complex (Amersham) diluted 1/250 in blocking buffer was applied for 1 1/2h at room temperature. Afterwards, the filters were washed three times in Tween-PBS and once in PBS, The filters were then incubated in PBS containing 0.0.5% (w/v) diaminobenzidine and 0.01% (v/v) hydrogen peroxide until background staining developed.

It should be clear that the formation of an immunological complex between the monoclonal antibody and the antigen is not limited to the precise conditions described above, but that all techniques that respect the immunochemical properties of the antibody and antigen binding will produce similar formation of an immunological complex.

The "histiocytes" and the "macrophages of the central nervous system" are defined in Greenfield's Neuropathology, and other classical textbooks on neuropathology.

A monoclonal antibody of the invention is characterized by the fact that it forms an immunological complex with the syngeneic polyclonal anti-idiotypic serum, particularly the monoclonal anti-idiotypic antibody, raised against the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG) and by the combination of the following properties:

- it forms an immunological complex with a non-phosphorylated structural epitope of an antigen belonging to activated microglial cells of the central nervous system and released from a sonicated NFT preparation, itself isolated from the cerebral cortex obtained from a patient having Alzheimer's disease,
- it forms an immunological complex with histiocytes and macrophages in the vicinity of necrosis areas in the central nervous system.

A particular preferred monoclonal antibody is of the IgM class, kappa type, or of the IgG, kappa type.

Advantageously, the monoclonal antibodies of the invention have the following properties:
- they do not bind immunologically with resting microglial cells of the central nervous system in pathological conditions,
- they do not bind immunologically with resting macrophages of organs other than the central nervous system,
- they do not bind immunologically with neurofibrillary tangles or amyloïd,
- they do not bind immunologically with endothelial cells or astrocytes, whereas the RCA-1 forms an immunological complex with said astrocytes under the same conditions,
- they do not bind immunologically with microglial cells of the healthy central nervous system, under the experimental conditions defined above.

The expressions "resting microglial cells of the central nervous system" and "astrocytes" are defined in e.g. Greenfield's Neuropathology, or other classical textbooks of neuropathology and neurology. The expression "the pathological conditions" refers to any disease affecting the central nervous system, and leading to metabolic and/or structural changes therein. The "neurofibrillary tangles" and "amyloid" are defined in: Selkoe DJ. Altered structural proteins in plaques and tangles: what do they tell us about the biology of Alzheimer's disease.

Neurobiol. Aging 1986; 7:425-432). The RCA1 is a lectin which recognizes endothelial cells (Mannoji H., H. Yeger, L.E. Becker (1986) A specific histochemical marker (lectin Ricinus communis agglutinin-1) for normal human microglia and application to routine histopathology. Acta Neuropathol (Berl) 71, 341-343).

Advantageously, the monoclonal antibodies of the invention do not bind immunologically with the microglial cells of normal brains; they do not bind immunologically with the normal microglial cells in the normal parts of a diseased brain; they bind immunologically with "activated" microglial cells in the abnormal parts of a diseased brain.

Advantageous monoclonal antibodies of the invention have the following additional properties:
- they bind immunologically with the multinuclear giant cells, characteristic of AIDS,
- they bind immunologically with rod cells from the brain of a neurosyphilis patient,
- they bind immunologically with cells from the brain of patients having Pick's disease, or amyotrophic lateral sclerosis, or Parkinson dementia complex of Guam,
- they bind immunologically with histiocytes in the case of a malignant cerebral hystiocytosis.

The expression "bind" means that the antibody forms an immunologically complex with the abovesaid antigen under the conditions described above.

The "multinuclear giant" cells are defined in Vazeux R., N. Brousse, A. Jarry, D. Henin, C. Marche, C. Vedrenne, J. Mikol, M. Wolff, C. Michon, W. Rozenbaum, J.F. Bureau, L. Montagnier, M. Brahic, (1987) AIDS subacute ecephalitis. Identification of HIV-infected cells. Am J. Pathol 126, 403-410. The "rod cells from the brain of a neurosyphilis patient", the "cells from brain of a patient having Pick's disease", the "cells from brain of a patient having amyotrophic disease", the "cells from a patient having Parkinson dementia complex of Guam", and the histiocytes in case of malignant cerebral histiocytosis are defined in Greenfield's Textbook of Neuropathology, Davis and Robertson, Textbook of Neuropathology, and other classical textbooks on neuropathology and neurology.

A preferred monoclonal antibody of the invention is secreted by the hybridoma deposited at the C.N.C.M. under n° I-881 on July 5, 1989 (AMC30 IgM).

A preferred monoclonal antibody of the invention is secreted by the hybridoma deposited at the C.N.C.M. under n° I-882 on July 5, 1989 (AMC30 IgG).

The invention also relates to monoclonal antibodies which form:
- immunological complexes with an antigen released from a sonicated NFT preparation, isolated from the cerebral cortex of a patient having Alzheimer's disease, which antigen also itself forms an immunological complex with the monoclonal antibody secreted by the hybridoma deposited at the C.N.C.M., under n° I-881 on July 5, 1989 (AMC30 IgM) or by the hybridoma deposited at the C.N.C.M. under n° I-882 on July 5, 1989 (AMC30 IgG).

The monoclonal antibodies of the invention advantageously do not bind immunologically with
- normal macrophages of organs different from the central nervous system,
- neurofibrillary tangles or amyloïd, endothelial cells or astrocytes, whereas the RCA-1 forms an immunological complex with said cells under the same conditions,
- microglial cells in the normal central nervous system.

The conditions under which the monoclonal antibodies of the invention do not bind immunologically with the above mentioned elements are identical to the conditions of light and electron immunomicroscopy described above.

The invention also relates to the hybridomas secreting the monoclonal antibodies above defined.

The above mentioned monoclonal antibodies are obtained by a process involving the obtention and isolation of hybridomas secreting the monoclonal antibodies.

A process for obtaining the hybridomas comprises:
- starting from spleen cells of an animal, e.g. mouse or rat, previously immunized in vivo or from spleen cells of such animals previously immunized in vitro with an antigen recognized by the monoclonal antibody secreted by the hybridoma deposited at C.N.C.M. under n° I-881 on July 5, 1989 (AMC30 IgM) or by the hybridoma deposited at the C.N.C.M. under n° I-882 on July 5, 1989 (AMC30 IgG),
- fusing said immunized cells with myeloma cells under hybridoma forming conditions and
- selecting those of the hybridomas which secrete the monoclonal antibodies which specifically recognize a non-phosphorylated structural epitope of the abovesaid antigen and which form an immunological complex with histiocytes and macrophages of the central nervous system in pathological conditions.

A process for producing the corresponding monoclonal antibodies comprises
- culturing the selected hybridomas as indicated above in an appropriate culture medium and
- recovering the monoclonal antibodies excreted by said selected hybridomas, or alternatively
- implanting the selected hybridomas into the peritoneum of a mouse and, when ascites have been produced in the animal,
- recovering the monoclonal antibodies then formed from said ascites.

The monoclonal antibodies of the invention can be prepared by in vitro conventional techniques such as cultures of immobilized cells using e.g. hollow fibers or microcapsules or such as cultures in homogenous suspension using e.g. airlift reactors.

In vitro immunization of the spleen cells is advantageous because the formation of the monoclonal antibodies is faster. The invention also relates to antigens which form an immunological complex with the monoclonal antibodies of the invention.

The antigen of the invention, which can be obtained from NFT preparations isolated from the cerebral cortex obtained from a patient having Alzheimer's disease and releasable therefrom by sonication thereof, is characterized by its capability of forming an immunological complex with a monoclonal antibody as defined above, advantageously with the monoclonal antibody secreted by the hybridoma deposited at the C.N.C.M., under n° I-881 on July 5, 1989 (AMC30 IgM) or by the hybridoma deposited at the C.N.C.M. under n° I-882 on July 5, 1989 (AMC30 IgG).

An advantageous antigen of the invention is characterized in that it is not expressed in normal bone marrow, lymph node, lung or spleen monocytes or macrophages.

The antigen of the invention is advantageously contained in the brain in the cerebro spinal fluid or the serum of a patient having Alzheimer's disease, AIDS or any brain infection or brain tumor involving activated microglia cells and it gives an immunological reaction with any monoclonal antibody of the invention.

The antigen according to the invention can be obtained by means of a process which comprises
- contacting one of the monoclonal antibodies of the invention with a sonicated preparation of NFT isolated from a patient having Alzheimer's disease, or rather from a diseased patient having had Alzheimer's disease under conditions suitable for producing an antigen-antibody complex,
- separating the antigen from said complex and recovering the antigen sought in a purified form.

Advantageously, the monoclonal antibodies used are in an immobilized state on a suitable support such as a resin SEPHAROSE (registered trade mark). The antigen can then be obtained upon
- bringing into contact with said monoclonal antibody the supernatant containing proteins and polypeptides as obtained as a result of an extraction procedure from sonicated NFT preparations with a RIPA buffer,

- washing the immobilized antibody-antigen complex then formed,
- treating that complex with a solution (e.g. 3M potassium thyocyanate, 2.5M magnesium chloride, 0.2M citrate-citric acid, pH 3.5 or 0.1M acetic acid) capable of producing the dissociation of the antigen-antibody complex and
- recovering the antigen in a purified form.

RIPA-buffer is 0.01 M Tris-HCl, pH 7.4, 0.15M NaCl, 1% sodium desoxycholate, 1% triton X100, 0.1% sodium dodecyl sulfate (SDS), 1mM phenyl-methyl-sulfonyl-fluoride (PMSF) (=0.5% of a 200mM PMSF solution in ethanol) and 500 kappa I.U. aprotinine/ml.

The invention also relates to the syngeneic polyclonal anti-idiotypic serum, and to the anti-idiotypic monoclonal antibodies, raised against the monoclonal antibodies of the invention.

More particularly, the invention relates to the syngeneic polyclonal anti-idiotypic serum, and to the anti-idiotypic monoclonal antibody, raised against the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG).

Monoclonal anti-idiotypic antibodies raised against the monoclonal antibodies of the invention can be used to replace the antigen of the invention when they correspond to the internal image of said antigen, for full disclosure on "internal image", see e.g. de Préval C. Immunoglobulins, pp. 144-219 in Bach J.F.; Immunology, Publ. Wiley and Sons, New York, 1978 or Fleischmann J.B., Davie J.M.: Immunoglobulins; allotypes and idiotypes, pp. 205-220 in Paul W.E.; Fundamental Immunology, Publ. Raven Press, New York, 1984; and Jerne, N.K.: Towards a network theory of the immune system Ann. Immunol. (Inst. Pasteur) 125C: 373-389 (1974).

The process of the invention for the detection or diagnosis in vitro of brain disease or brain infection involving activated microglial cells, e.g. Alzheimer's disease, includes:
- contacting in vitro a cell preparation from a patient suspected of suffering of brain disease or brain infection involving activated microglial cells, more particularly Alzheimer's disease, with a monoclonal antibody of the invention under conditions suitable for producing an antigen-antibody complex and
- detecting the immunological binding of said antibody to said cell preparation.

The cell preparation can be produced in any appropriate manner, e.g. from a biopsy obtained from that patient, particularly after sonication thereof and, whenever appropriate, fixing in a suitable medium, e.g. 4% formalin.

The detection of the immunologically bonded monoclonal antibody can be achieved by conventional technology. Advantageously, the monoclonal antibody of the invention carries itself a marker or a group for direct or indirect coupling with a marker as examplified hereafter.

A particularly advantageous embodiment of the process of the invention comprises contacting a serum preparation (containing the corresponding antigen) obtained from the patient to be diagnosed with the monoclonal antibody of the invention.

The invention also relates to a kit for the diagnosis in vitro of one of the following diseases: Alzheimer's disease, encephalitis induced by AIDS virus, and other diseases with pronounced activation of the microglial cells of the central nervous system, characterized in that it comprised:
- at least a micro-plate for deposition thereon of the biological sample to be diagnosed in vitro ,
- a preparation containing one of the monoclonal antibodies of the invention,
- a marker either for specific tagging or coupling with said monoclonal antibody,
- appropriate buffer solutions for carrying out the immunological reaction between a test sample and said monoclonal antibody on the one hand, and the bonded monoclonal antibodies and the marker on the other hand.

The invention also relates to a kit, as described above, also containing a preparation of the antigen of the invention, said antigen of the invention being either a standard (for quantitative determination of the antigen which is sought) or a competitor, with respect to the antigen which is sought, for the kit to be used in a competition dosage process.

The invention also relates to a kit, as described above, also containing a monoclonal anti-idiotypic antibody of the invention, particularly the one corresponding to the internal image of the antigen of the invention, said monoclonal anti-idiotypic antibody of the invention being either a standard (for quantitative determination of the antigen which is sought) or a competitor, with respect to the antigen which is sought, for the kit to be used in a competition dosage process.

The invention also relates to corresponding nucleic acids coding for the antigen of the invention, particularly that which is recognized by the antibody secreted by the hybridomas deposited at the C.N.C.M. under n° I-881 on July 5, 1989 (AMC30 IgM) or by the hybridoma deposited at the C.N.C.M. under n° I-882 on July 5, 1989 (AMC30 IgG).

Different methods are of course available to the man skilled in the art to produce or isolate such nucleic acids, particularly (but not necessarily) when the aminoacid sequence of the antigen of the invention becomes available, at least in part, as a result of conventional peptide sequencing procedures.

One of such methods comprises:
- contacting under hybridization conditions, a fragmented DNA composition obtained from NFT cells
with probes consisting of nucleic acid sequences
corresponding to selected amino acid sequences of
the antigen of the invention,
- recovering the hybrids formed,
- then separating under suitable denaturing conditions, the DNA strand from the starting probes,
said DNA strand then containing at least part of the
nucleic acid sequence coding for said antigen.

The process may then further comprise
sequencing such strand and after comparison with
the possible theoretical nucleotide sequences likely
to be in correspondance with the previously determined aminoacid sequences of the antigen of the
invention, identifying the relevant part of the nucleic
acid.

Another preferred method, more practical and
making that nucleic acid more readily accessible,
even without preliminary sequencing of at least part
of the amino acid sequence of the antigen, comprises:
- recovering the messenger RNAs from cells of the
cerebral cortex of a patient having had Alzheimer's
disease,
- forming a library cDNA by reverse transcription of
said mRNAs,
- optionally fragmenting said cDNAs, e.g. with a
SaulIIA restriction enzyme,
- inserting them in suitable cloning vectors under
the control of suitable promoters and regulating
elements authorizing the later expression in competent cellular hosts of the open reading frames
contained in the inserts then contained in such
vectors,
- transforming the appropriate competent cellular
hosts with the modified vectors containing such
cDNAs or cDNA fragments, and recovering the
transformed cell colonies,
- contacting the expression products of said transformed cell colonies, if need be after the lysis of
said cells with a monoclonal antibody according to
the invention, under conditions authorizing the production of an immunological complex between said
antibody and the corresponding antigen and
- screening these of the cells which produce proteins recognized by the monoclonal antibody of the
invention,
- recovering, from the selected cells, the foreign
DNA fragment responsible for the expression product which had been recognized by the monoclonal
antibody,
- optionally sequencing and identifying that particular nucleic acid and
- finally, after having identified open reading frame which was responsible for the production of the
antigen recognized by the monoclonal antibody,
recovering a nucleic acid fragment which contained
that open reading frame.

Needless to say that this method also provides
a method which allows for a determination of the
amino acid sequence of at least part of the basic
polypeptide structure of the antigen of the invention.

The nucleic acids obtained or fragments thereof can then in turn be used for the production of
the antigen of the invention containing sites specifically recognized by the starting monoclonal antibodies, e.g. after insertion of said nucleic acids or
fragments in appropriate vectors and transformation
of a competent organism with the modified vector
to produce the antigen under consideration. Needless to say that under such circumstances, the
particular nucleic acid fragment must be inserted
again in that vector under the control of a suitable
promoter and followed by a suitable termination
signal in order to provide for the production of that
antigen in the competent host.

The nucleic acid obtained or part thereof can
also be used for the production of probes, particularly after radioactive labeling or modification by
conventional procedures in order to make them
later detectable by suitable markers. Advantageous
probes of the invention for detecting the presence
of an antigen above defined which comprises a
sequence belonging to the nucleic acids above
defined or the corresponding complementary nucleic acid, and which includes itself from 10 to the
maximum number of nucleotides of said nucleic
acids. In turn, these probes can be used for the
detection of the nucleic acid sequences encoding
the antigen responsible for Alzheimer's disease or
the other diseases referred to in the present application. These probes may either consist of the
nucleic acid or part thereof in an isolated form.
They may also consist of a recombinant DNA containing said nucleic acid or part thereof, it however
being understood that care should then be taken
that the other parts of such recombinant DNA
probe are not likely to hybridize with the nucleic
acid compositions obtained from cell preparations
originating from patients to be diagnosed.

Such additional in vitro diagnosis methods then
comprise:
- starting from a cell preparation obtained from the
cerebral cortex of a patient suspected of suffering
brain disease or brain infection involving activated
microglial cells, e.g. Alzheimer's disease, whose
nucleic acids have been made accessible to possible hybridization with other nucleic acids, whenever
required,
- contacting said cell preparation or the nucleic
acids previously extracted therefrom with the above defined probe under suitable hybridization conditions, which possibly provide for the production of an hybrid between said probe and a sequence coding for said antigen and
- detecting the hybrid formed, if any, and assessing the possible existence or to the contrary absence of such neurofibrillar degeneration in said patient depending upon the occurence or not of hybridization.

Preferred additional characteristics of the invention will appear in the course of the following description of preferred conditions under which hybridomas and corresponding monoclonal antibodies of the invention can be obtained, which antibodies can then give access to all the other components mentioned above according to procedures which, as such, are all accessible to a person skilled in the art.

EXAMPLE I : Preparation of the monoclonal antibody AMC30 (IgM, kappa type):

**Isolation of NFT for immunization:**

NFT were isolated by the long (Iqbal K., Zaïdi T, Thompson CH, et al. Alzheimer paired helical filaments bulk: isolation, solubility and protein composition. Acta Neuropath. (Berl.) 1984; 62: 167-177) procedure from the cerebral cortex obtained at autopsy from a patient with apparently sporadic, neuropathologically confirmed Alzheimer's disease. The NFT preparation was verified by polarized light microscopy after Congo Red staining. This showed flame shaped congophilic NFT, other congophilic material, cell nuclei, capillaries and amorphous debris. The NFT preparation was sonicated before immunization.

**In vitro immunization and fusion procedure:**

Spleen cells from unimmunised BALB/c mice were cultured in 50% thymocyte conditioned HB 101 medium (Hana Biologicals) with 20% fetal calf serum in the presence of 40μg/ml sonicated NFT preparation. On the third, fourth and fifth day, $10^8$ spleen cells were mixed with 2 x $10^7$ P3/NS-1/1Ag4-1 myeloma cells and fused in 25% polyethylene glycol for 8 minutes as described (Koprowski H, Gerhard W, CM. Production of antibodies against influenza virus by somatic cell by somatic cell hybrids between mouse myeloma and primed spleen cells. Proc. Natl. Acad. Sci. USA 1977; 74: 2985-2988). After each of the three fusions a series of 1440 cultures each containing 5 x $10^4$ cells from the fusion mixture and $10^5$ unimmunised BALB/c

spleen cells in 0.2 ml was established in 96-well plates (Nunc) in complete HAT selective medium. HAT selective medium was HB101 medium supplemented with 15% heat-inactivated fetal calf serum and containing hypoxanthine, aminopterine and thymidine. Specific antibody secreting cultures were expanded to 2.5 ml and cloned by limiting dilution.

**Specific antibody screening:**

**a) Material and methods:**

The solid-phase radioimmunoassay to screen hybridoma supernatants for antibody production went as follows. A semi-purified NFT preparation, consisting of the final pellet of the long Iqbal procedure was sonicated and diluted to approximately 10μg protein/ml in 0.1 M sodium bicarbonate. This antigen was adsorbed to polyvinyl chloride flat-bottom 96-well plates (Falcon) as described. Fifty μl of hybridoma supernatant was applied to each well. After 4 h at 4°C, the wells were washed three times with 0.1 M sodium phosphate buffer (pH 7.4) containing 0.15 M sodium chloride (PBS) and 2% fetal calf serum, and incubated with 50 μl $I^{125}$ labeled anti-mouse immunoglobulin (25.000 cpm) (Amersham). After 2h, the wells were washed three times, cut from the plate with a hot wire and counted for 1 min in a gamma counter. A sample was considered positive when the cpm bound by it were greater than the mean plus two standard deviations of the cpm bound by complete HAT medium.

**b) Results:**

Hybridoma cell growth occured in 829 of the 4320 cultures. Twenty three stable antibody secreting hybridomas were identified with the screening assay. The yield of specific hybridomas was the same for each fusion, irrespective of the duration of in vitro antigen priming. One of these monoclonal antibodies, termed AMC30, has the above mentioned properties. It is unambiguously of the IgM class, kappa type.

**Light immunomicroscopy:**

**a) Material and method:**

Brain tissue of apparently sporadile (N = 4) and senile (N = 4) AD, autosomal dominantly inherited AD in two large Belgian families (N = 14) (Van Broeckhoven C., A.M. Genthe, A. Vandenberghe, B. Horsthemke, H. Backhovens, P. Raeymaekers, W. Van Hul, A. Wehnert, J. Gheuens, P. Cras, M. Bruyland, J.J. Martin, M. Salbaum, G. Multhaup, C.L. Masters, K. Beyreuther, H.M.D. Gurling, M.J. Mullan, A. Holland, A. Barton, N. Irving, R. Williamson, S.J. Richards, J.A. Hardy (1987) Failure of familial Alzheimer disease to segregate with the A4-amyloid gene in several European families. Nature 329, 153-155), Parkinson dementia complex (N = 3) and amyotrophic lateral sclerosis (N = 2) of Guam, a person from Guam with NFT but without neurological disease, Pick's disease (N = 3), post-encephalitic (N = 1) and idiopathic (N = 1) Parkinsonism, progressive supranuclear paralysis (N = 3), subacute sclerosing panencephalitis (N = 1), cerebellar astrocytoma with Rosenthal fibers (N = 1), ganglioma (N = 2), sudden infant death syndrome (N = 2) was taken at autopsy. Cerebral cortex of patients obtained at surgery for brain tumor (N = 5) and normal age-matched controls (N = 3) were used as controls. Control human spinal cord and peripheral nerve, rat sciatic nerve and other human tissues (adrenal, lung, liver, skeletal muscle, kidney, ovary, pancreas, spleen) were also studied. Each tissue sample was fixed by immersion in 4% formalin and embedded in paraffin. Four μm thick sections were prepared.

The AMC30 monoclonal antibody of the invention (hybridoma ascites in a dilution of 1:10.000 to 1:20.000), or purified AMC30 IgM (0.01 mg/ml) was applied either with the avidin biotinylated peroxidase complex technique (Hsu SM, Raine L, Fanger H. Use of avidin-biotin complex (ABC) in immunoperoxydase techniques. J. Histochem. Cytochem. 1981; 29: 577-580), or with soluble peroxidase-anti-peroxidase complex technique (Sternberger LA. Immunocytochemistry (3rd ed.) Wiley, New York, 1986). To minimize non-specific staining, the sections were first treated with normal human serum, diluted 1 : 100, as described (Perentes and Rubinstein - preincubation with NHS to prevent non-specific staining of astrocytes). Diaminobenzidine was used as chromogen. The myeloma protein MOPC104E (IgM) (Litton Bionetics) and the NFT200 monoclonal antibody (IgM), which is directed against paired helical filaments (Gheuens et al., submitted), served as control. A monoclonal anti-GFAP (anti-glial fibrillary acidic protein) antibody (Gheuens et al., Identification of several forms of the glial fibrillary acidic protein, or α-albumin, by a specific monoclonal antibody. J. Neurochem. 1984; 964-970) was used to identify astrocytes. Conventional cresyl violet, Holzer, Spielmeyer, Bodian, Von Braunmuhl, PAS and Congo Red stainings were also done. The Congo Red sections were examined in polarised light.

**b) Results:**

- Alzheimer's disease:

Immunolabeled process bearing cells were localized in senile plaques in the cerebral cortex of all presenile and senile, sporadic and familial patients with Alzheimer's disease. The morphology of these immunolabeled cells fits the classical description of microglial cells. They had an elongated and sometimes irregular nucleus, and a number of fine ramifications.

Similar cells were distributed throughout the cortex, preferentially surrounding blood vessels. Rare perivascular histiocytes were also stained. Very rare immunolabeled cells were found in the subcortical white matter, but the deep white matter was completely devoid of any labelling. Sometimes a faint nuclear staining was observed. Neurofibrillary tangles and vascular amyloid deposits remained negative. Astrocytes, as identified with anti-GFAP monoclonal antibody, were not labelled, nor were endothelial cells.

- AIDS:

The multinuclear giant cells, characteristic of AIDS encephalitis, were labeled, allthough less intensely than the microglia in the surrounding tissue. In the vicinity of necroses, numerous macrophages and foam cells were stained.

- Neurosyphilis:

In a stereotactic brain biopsy of a serologically confirmed neurosyphilis patient with multifocal abnormal signal on MRI, numerous rod cells were stained by the monoclonal antibody AMC30. These cells were preferentially located in the cortex. Microglial nodules were not present.

- Pick's disease, Amyotrophic lateral sclerosis, Parkinson dementia complex of Guam:

Some predominantly perivascular and rounded cells were labelled in all these conditions. Cells with extensions were extremely rare.

- Brain tumor biopsies:

In low or high grade astrocytomas, gangliogliomas or glioblastomas, no cells were recognized. In an oligodendroglioma, rare abnormal oligodendrocytes were labelled. In the parenchyma adjoining infiltrating tumors, no cells were stained. In a case of malignant cerebral histiocytosis, numerous abnormal histiocytes were labelled. The staining was cytoplasmic and granular.

- Control central nervous system:

In aged, juvenile or neonatal brain of patients who died from accidents or non-neurologic illnesses of brief duration, microglial cells remained unlabeled.

- Other normal organs:

No staining of Kupffer cells in the liver, Langerhans cells in the skin, alveolar macrophages in the lung, bone marrow and spleen or lymph node histiocytes or monocytes.

- Lesions in organs outside the nervous system:

In a malignant fibrous histiocytoma, repeated biopsies showed labelling of macrophages and abnormal spindle cells with monoclonal antibody of the invention, with $\alpha$1-antichymotrypsin and with RCA-1. In granulomatous inflammations, tuberculosis and sarcoidosis, some macrophages and epitheloid cells were stained by all three probes. RCA demonstrated the highest amount of macrophages. The giant cells of Langerhans type were labelled with the monoclonal antibody and RCA, but remained negative with $\alpha$1-antichymotrypsin. In a dermic histiocytoma, fibroblasts and histiocytes were labelled with $\alpha$1-antichymotrypsin, but were not immunoreactive with monoclonal antibody or RCA.

Some Langerhans cells in the epidermis were stained with RCA. In a juvenile xanthogranuloma, macrophages and spindle cells were only labelled with RCA. Macrophages in portal fields and in the liver parenchyma surrounding a metastasis were decorated with all three probes. Faint nuclear staining with the monoclonal antibody was encountered in several cases.

**Ricinus communis lectin stainings :**

**a) Material and methods :**

For staining with lectins, a technique similar to the above can be used (Mannoji H., H. Yger, L.E. Becker (1986) A specific histochemical marker (lectin Ricinus communis agglutinin-1) for normal human microglia and application to routine histopathology. Acta Neuropathol (Berl) 71, 341-343). After deparaffination and inhibition of endogenous peroxidase and non-specific protein binding sites, biotinylated Ricinus communis agglutinin (RCA-1) (Sigma) was applied to the section. Adequate washing was followed by incubation with streptavidinbiotinylated horse radish peroxidase complex. The final incubation was with diaminobenzidine. Preincubation with lactose abolishes all labelling of microglia.

**b) Results :**

In all instances did RCA-1 label cells comparable to those that were labeled with the AMC30 monoclonal antibody, and that conformed the classical morphological criteria for microglial cells. However, RCA-1 also stained endothelial cells and some astrocytes, that remained negative with the AMC30 monoclonal antibody.

**Dephosphorylation experiments:**

**a) Material and methods:**

To assess if AMC30 monoclonal antibody was directed against a phosphorylated epitope, semipurified NFT were coated to PVC-plates, and then treated overnight at 37° C with 1 IU of type III E.coli alkaline-phosphatase in 100 ml 0.1 M Tris buffer, pH 8.0, containing 0.01 M phenyl-methyl-sulfonyl-fluoride. Next, AMC30 monoclonal antibody was applied, and an avidine-biotinylated peroxidase complex (Amersham) ELISA procedure was done, using 3,3',5, 5'-tetramethyl benzidine (TMB) (Boehringer) as substrate. GFAP (antiglial fibrillary acidic protein) and monoclonal anti-GFAP, as well as neurofilament proteins and monoclonal antineurofilament antibody served as controls. The monoclonal anti-GFAP was directed against a non-phosphorylated epitope, the monoclonal antineurofilament antibody was directed against a phosphorylated epitope (Gheuens et al. unpublished results).

**b) Results :**

Semi-purified NFT that were treated with alkaline-phosphatase retained their reactivity with AMC30 monoclonal antibody. This indicated that AMC30 was not directed against a phosphorylated epitope.

**Other methods:**

Class and type determination of the monoclonal antibody was done with class- and type-specific anti-mouse immunoglobulin reagents (Litton Bionetics) in Ouchterlony double immunodiffusion experiments. Sephacryl S300 (Pharmacia) gel filtration chromatography was used to purify the AMC30 monoclonal

EXAMPLE II : Preparation of a switch variant monoclonal antibody (of the IgG, kappa type) from the AMC30 hybridoma culture.

1) Selection of an $IgG_1$, kappa type secreting switch variant from the AMC30 hybridoma culture:

The AMC30 hybridoma secretes monoclonal antibody of the IgM class, kappa type. However, IgM-monoclonal antibodies can offer technical disadvantages over IgG-monoclonal antibodies in immunochemical application. We have therefore devised a method by which it was possible to select and clone a variant that secretes $IgG_1$, kappa with identical antigenic specificity as the IgM, kappa AMC30 monoclonal antibody from an AMC30 hybridoma culture. Such so-called "switch-variants", that secrete antibody of a different class, are known to occur spontaneously in IgM-secreting hybridomas. However, such events are rare, and estimated to occur at a frequency of only $10^{-5}$ to $10^{-8}$. The desired switch-variant therefore has to be identified, selected and cloned from the IgM-producing hybridoma quickly, before overgrowth occurs.

The methods that was used went as follows. A series of 480 cultures, each containing $10^3$ AMC30 hybridoma cells in 0.15 ml complete medium was established in five 96-well plates. Complete medium was Dulbecco's Modified Eagle Medium, with high glucose (4.500 mg/L), supplemented with 1.2 mM sodium pyruvate, 2 mM glutamine, 100 I.U./ml penicilline, 100 I.U./ml streptomycine and 10 % heat-inactivated (30 minutes at 56°C) fetal calf serum. After incubation for 5 days at 37°C in a 5 % $CO_2$ atmosphere, each culture was screened for $IgG_1$ production (as described hereafter). Positive cultures were plated out at 200 cells in 0.15 ml complete medium in 96-well plates, and screened for $IgG_1$ production after 5 days. The cultures that secreted $IgG_1$ were then cloned in three series of 0.1 ml cultures, respectively containing 100, 10 and 0.5 cells per well, and each containing $10^5$ BALB/c thymocyte feeder cells. After 6 days the wells in which cell growth had occurred were screened for the presence of $IgG_1$ production and the absence of IgM production. The $IgG_1$ positive, IgM negative cultures were then recloned as described above at 0.3 cells/well, and tested again after 7 days. An $IgG_1$ secreting switch-variant of the AMC30 hybridoma was thus isolated. The antibody was tested for antigen specificity as described for the AMC30 monoclonal antibody. The results indicated that the $IgG_1$ switch-variant monoclonal antibody had the same antigen-specificity as the IgM AMC30. It was also demonstrated that the $IgG_1$ switch-variant monoclonal antibody expressed the same idiotype as the IgM monoclonal antibody (as described hereafter).

**2) Screening assay for detection of $IgG_1$ secretion by hybridoma cultures.**

Polyvinyl chloride flat-bottom 96-well plates (Nunc F16 Maxisorp) were coated overnight at 4°C with 100 $\mu$l polyclonal rabbit anti-mouse $IgG_1$ serum (Organon), diluted 1:1000 in 0.01 M sodium chloride -0.01 M sodium azide - 0.01 M Tris buffer, pH 8.5. All subsequent incubations were at 37°C. After two washes with PBS containing 0.05 % Tween 80 (Tween-PBS), the plates were saturated for 1 h with 150 $\mu$l 10 % casein in PBS (casein-PBS). Next the plates were washed three times with Tween-PBS, and 100 $\mu$l of the supernatant to be tested was added. After 1 h, the plates were washed again, and 100 $\mu$l of biotinylated goat anti-mouse $IgG_1$ serum (Amersham), diluted 1:2000 in casein-PBS was added to each well. After 1 h, the plates were washed three times, and incubated with 100 $\mu$l streptavidine-biotinylated peroxidase complex (Amersham RPN 1051), diluted 1:1000 in casein-PBS for 30 minutes. Next, the plates were washed four times with Tween-PBS, and 100 $\mu$l 0.0004 M 3,3',5,5'-tetramethylbenzidine (TMB) (Boehringer 784974) in 0.1 M phosphate-citrate buffer, pH 4.3, containing 0.004 % hydrogen peroxide was added to each well. After 30 minutes at 37°C, the reaction was stopped by adding 50 $\mu$l 2 M sulfuric acid to each well. Absorption was then read at 450 nm.

To detect IgM secretion by the hybridomas, the plates were coated with polyclonal rabbit anti-mouse IgM serum and biotinylated goat anti-mouse IgM serum was used as second reagent. Otherwise, the screening assay was identical to that described above.

EXAMPLE III : Preparation of syngeneic monoclonal anti-idiotypic antibodies to AMC30:

The AMC30 IgM, kappa monoclonal antibody, as well the $IgG_1$ kappa monoclonal antibody that is

secreted by an IgG$_1$, kappa switch variant of the AMC30 hybridoma (see preceding paragraph) are defined through their idiotype, as described earlier

1) Production of syngeneic anti-idiotype sera to the monoclonal antibody AMC30.

AMC30 monoclonal antibody was purified from AMC30 hybridoma ascites by gel filtration FPLC over Sephacryl S-300 (column C26,L:86 cm). The sample was 10 ml AMC30 hybridoma ascites, diluted 1:2 in 0.08 M phosphate buffer with 0.077 M sodium chloride. The flow rate was 10 ml/h. The AMC30 monoclonal antibody appeared in the void volume. The purified AMC30 was then concentrated to 1 mg/ml in saline. BALB/c mice, 10 to 12 weeks of age, were immunised as follows. Purified AMC30 was conjugated to keyhole limpet hemocyanin (KLH) (Calbiochem) (1 mg/ml in saline) with glutaraldehyde as described in the paper by Bona et al. The resulting AMC30-KLH conjugate was emulsified in complete Freund adjuvant to give 75 $\mu$g of AMC30-KLH conjugate and 100 $\mu$g of Mycobacterium tuberculosis H37 Ra (Difco) in 100 $\mu$l of emulsion. Each mouse was given 50 $\mu$l of this emulsion intraperitoneally, and another 50 $\mu$l was distributed among the hind legs footpads and the inguinal and axillary regions. Five days after the first immunization, 75 $\mu$g of AMC30-KLH conjugate in 100 $\mu$l of incomplete Freund adjuvant was injected over the axillary and inguinal regions. The mice were boosted once weekly with 75 $\mu$g of conjugate in saline given in the axillary and inguinal regions. The mice were first bled 6 days after injection 6 and subsequently every 2 weeks. The anti-idiotypic nature of the syngeneic antisera is correlated to immunological specificity of the antiserum to AMC30, and products derived from it, but not to other BALB/c immunoglobulins of the IgM class, kappa type, nor any other BALB/c immunoglobulin. This is done using appropriate ELISA procedures, or agglutination assays with immunoglobulin-coated sheep red blood cells, as described in the papers under reference.

## 2) Production of syngeneic monoclonal anti-idiotypic antibodies to AMC30.

Spleen cells, taken from a mouse that has been immunised with AMC30-KLH conjugate as described in the preceding paragraph, can be fused to BALB/c myeloma cells in the manner described elsewhere in this application, to produce hybridoma cell cultures. The hybridoma cultures can be screened for the production of antibodies to AMC30 monoclonal antibody using the multivalent antibody radioimmunoassay, that has been described before (Gheuens J., McFarlin D.E.: Multivalent antibody radioimmunoassay (MARIA) for screening specific antibody secretion by lymphocyte hybridoma cultures. Meth. Enzymol. 121: 425-433, 1986). The anti-idiotypic nature of such monoclonal antibodies to AMC30 could be assessed as described in the papers under reference. The anti-idiotypic nature of antibodies is correlated to the immunological specificity of such antibodies to AMC30, and products derived from it, but not to other BALB/c immunoglobulins of the IgM class, kappa type, nor any other BALB/c immunoglobulin.

## Claims

1. Monoclonal antibody which has the combination of the following properties:
- it forms an immunological complex with a non-phosphorylated epitope of an antigen belonging to activated microglial cells of the central nervous system and released from a sonicated NFT preparation, itself isolated from the cerebral cortex obtained from a patient having Alzheimer's disease,
- it forms an immunological complex with histiocytes and macrophages of the central nervous system.

2. Monoclonal antibody characterized by the fact that it forms an immunological complex with the syngeneic polyclonal anti-idiotypic serum, particularly with the monoclonal anti-idiotypic antibody, raised against the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG).

3. Monoclonal antibody characterized by the fact that it has the combination of the following properties:
- it forms an immunological complex with a non-phosphorylated epitope of an antigen belonging to activated microglial cells of the central nervous system and released from a sonicated NFT preparation, itself isolated from the cerebral cortex obtained from a patient having Alzheimer's disease,
- it forms an immunological complex with histiocytes and macrophages of the central nervous system and characterized by the fact that it forms an immunological complex with with the syngeneic polyclonal anti-idiotypic serum, particularly with the monoclonal anti-idiotypic antibody, raised against the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG).

4. Monoclonal antibody according to anyone of claims 1 to 3, which is of the IgM class, kappa type or of the IgG kappa type.

5. Monoclonal antibody according to anyone of claims 1 to 5, which:
- does not bind immunologically with resting microglial cells of the central nervous system in pathological conditions,
- does not bind immunologically with macrophages of organs different from the central nervous system,
- does not bind immunologically with neuro-fibrillary tangles or amyloïd,
- does not bind immunologically with endothelial cells or astrocytes, whereas the RCA-1 forms an immunological complex with said cells under the same conditions,
- does not bind immunologically with microglia cells of the healthy central nervous system.

6. Monoclonal antibody according to anyone of claims 1 to 5, which :
- binds immunologically with the multinuclear giant cells, characteristic of AIDS,
- binds immunologically with rod cells from the brain of a neurosyphilis patient,
- binds immunologically with cells from the brain of patient having Pick's disease, amyotrophic lateral sclerosis, Parkinson dementia complex of Guam,
- binds immunologically with histiocytes in the case of a malignant cerebral hystiocytosis.

7. Monoclonal antibody, which is secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG).

8. Monoclonal antibody, which
- forms an immunological complex with an antigen released from a sonicated NFT preparation isolated from the cerebral cortex of a patient having Alzheimer's disease, which antigen itself forms an immunological complex with the monoclonal antibody secreted by the hybridoma deposited at the C.N.C.M., under n° I-881 on July 5, 1989 (AMC30 IgM) or by the hybridoma deposited at the C.N.C.M. under n° I-882 on July 5, 1989 (AMC30 IgG).

9. Monoclonal antibody according to claim 8, which does not bind immunologically with :
- macrophages in organs other than the central nervous system,
- neuro-fibrillary tangles or amyloïd,
- endothelial cells or astrocytes, whereas the RCA-1 forms an immunological complex with said cells under the same conditions,
- microglia in the normal central nervous system.

10. Hybridoma, which secretes a monoclonal antibody according to anyone of claims 1 to 9.

11. Antigen, releasable by a sonicated NFT preparation, isolated from the cerebral cortex obtained from a patient having Alzheimer's disease and which forms an immunological complex with the monoclonal antibody of anyone of claims 1 to 9.

12. Antigen according to claim 11, which is not expressed in normal bone marrow, lymph node, lung or spleen monocytes or macrophages.

13. Antigen which is contained in the brain, in the cerebro spinal fluid or the serum of a patient having Alzheimer's disease, AIDS or any brain infection or brain tumor involving activated microglia cells and which forms an immunological complex with a monoclonal antibody of anyone of claims 1 to 9.

14. Antigen which forms an immunological complex with the monoclonal antibody secreted by the hybridoma deposited at the C.N.C.M., under n° I-881 on July 5, 1989 (AMC30 IgM) or by the hybridoma deposited at the C.N.C.M. under n° I-882 on July 5, 1989 (AMC30 IgG).

15. Monoclonal anti-idiotypic antibody characterized by that fact that it forms an immunological complex with anyone of the monoclonal antibodies of claims 1 to 9.

16. Monoclonal anti-idiotypic antibody characterized by that fact that it forms an immunological complex with the monoclonal antibody secreted by the hybridoma deposited at the C.N.C.M. under n° I-881 on July 5, 1989 (AMC30 IgM) or by the hybridoma deposited at the C.N.C.M. under n° I-882 on July 5, 1989 (AMC30 IgG).

17. Process for obtaining and isolating hybridomas secreting a monoclonal antibody according to anyone of claims 1 to 9, characterized in that it comprises :
- starting from spleen cells of an animal, e.g. mouse or rat, previously immunized in vivo or from spleen cells of such cells previously immunized in vitro with an antigen recognized by the monoclonal antibody deposited at C.N.C.M. under n° I-881 on July 5, 1989 (AMC30 IgM) or by the hybridoma deposited at the C.N.C.M. under n° I-882 on July 5, 1989 (AMC30 IgG)
- fusing said immunized cells with myeloma cells under hybridoma forming conditions and
- selecting those of the hybridomas which secret the monoclonal antibodies which specifically recognize a non-phosphorylated structural epitope of the antigen according to claim 11 to 14 and which forms an immunological complex with histiocytes and macrophages of the central nervous system.

18. Process for producing monoclonal antibodies according to claims 1 to 9 which comprises :
- culturing the selected hybridomas according to claim 8, in an appropriate medium culture and
- recovering the monoclonal antibodies excreted by said selected hybridomas
or alternatively
- implanting the selected hybridomas into the peritoneum of a mouse and when ascites have been produced into the animal recovering the monoclonal antibodies then formed from said ascites.

19. Process for the preparation of the antigen according to claims 11 to 14, which comprises
- starting from nucleic acids or fragments thereof coding for the antigen according to claims 11 to 14 and inserting said nucleic acids or fragments in an appropriate vector, under the control of a suitable promoter and followed by a suitable termination signal,
- transforming a competent organism with the modified vector to produce said antigen.

20. Process for the detection or diagnosis in vitro of brain disease or brain infection involving activated microglia cells, e.g. Alzheimer's disease, which comprises :
- contacting the monoclonal antibody according to anyone of claims 1 to 9, with a sonicated preparation of NFT isolated from a patient having had Alzheimer's disease under conditions suitable for producing an antigen-antibody complex and
- separating the antigen from said complex and recovering the antigen sought in a purified form.

21. Process for the detection or diagnosis in vitro of brain disease or brain infection involving activated microglia cells, e.g. Alzheimer's disease, which includes :
- contacting in vitro a cell preparation from a patient suspected of suffering of neurofibrillar degeneration, more particularly Alzheimer's disease, with a monoclonal antibody according to anyone of claims 1 to 9, under conditions suitable for producing an antigen-antibody complex and
- detecting the immunological binding of said antibody to said cell preparations.

22. Kit for the diagnosis in vitro , of one of the following diseases Alzheimer's disease, encephalitis induced by AIDS virus and other diseases with pronounced activation of the microglial cells of the central nervous system, which comprises :
- at least a micro-plate for deposition thereon of the biological sample to be diagnosed in vitro ,
- a preparation containing a monoclonal antibody of anyone of claims 1 to 9,
- labeled marker either for specific tagging or coupling with said monoclonal antibody,
- appropriate buffer solutions for carrying out the immunological reaction between a test sample and said monoclonal antibody on the one hand, and the bonded monoclonal antibodies and the marker on the other hand,
- possibly the antigen according to claims 11 to 14 or the monoclonal anti-idiotypic antibody according to claims 15 or 16, for standard purposes, or for competition purposes with respect to the antigen which is sought.

23. Nucleic acid sequence coding for the antigen according to claim 11 to 14.

24. Probe for detecting the presence of the antigen according to claim 11 to 14, which comprises :
- a sequence belonging to the nucleic acid of claim 23, or to the complementary nucleic acid of claim 23, and which includes itself from 10 to the maximum number of nucleotides of the nucleic acid of claim 23.

25. Process for the detection or diagnosis in vitro of brain disease or brain infection involving activated microglia cells, such as Alzheimer's disease which comprises
- starting from a cell preparation, particularly NFT obtained from the cerebral cortex of a patient suspected of suffering neurofibrillar degeneration, e.g. Alzheimer's disease, whose nucleic acids have been made accessible to possible hybridization with other nucleic acids, whenever required,
- contacting said cell preparation or the nucleic acids previously extracted therefrom with the above defined probe under suitable hybridization conditions, which possibly provide for the production of an hybrid between said probe and a sequence coding for said antigen and
- detecting the hybrid formed, if any, and assessing the possible existence or to the contrary absence of such neurofibrillar degeneration in said patient depending upon the occurence or not of hybridization.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | ACTA NEUROPATH., vol. 77, 1989, pages 569-575, Springer-Verlag; S. HAGA et al.: "Demonstration of micro-glial cells in and around senile (neuritic) plaques in the Alzheimer brain. An immunohistochemical study using a novel monoclonal antibody"<br>* The whole document * | 1-14, 17-25 | C 12 P 21/08<br>G 01 N 33/577<br>C 12 N 15/12<br>C 12 Q 1/68 |
| Y | IDEM | 15,16 | |
| D,Y | EUR. J. IMMUNOL., vol. 12, 1982, pages 701-703; J. GHEUENS et al.: "Use of monoclonal anti-idiotypic antibody to P3-X63Ag8 myeloma protein for analysis and purification of B lymphocyte hybridoma products"<br>* The whole document * | 15,16 | |
| D,X | ACTA NEUROPATHOL., vol. 62, 1984, pages 167-177; K. IQBAL et al.: "Alzheimer paired helical filaments: Bulk isola-tion, solubility, and protein composition"<br>* Page 171, column 2, line 4 - page 172, column 2, line 2 * | 11-14 | |
| A | JOURNAL OF NEUROPATHOLOGY AND EXPERIMENTAL NEUROLOGY, vol. 47, no. 6, November 1988, pages 579-587, American Association of Neuropathologists; J.M. MILES et al.: "A new immunoperoxidase marker for micro-glia in paraffin section" | | **TECHNICAL FIELDS SEARCHED (Int. Cl.5)**<br><br>C 12 P |

The present search report has been drawn up for all claims

| Place of search | Date of completion of search | Examiner |
|---|---|---|
| The Hague | 29 October 90 | SKELLY J.M. |

CATEGORY OF CITED DOCUMENTS
X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document